(19) Europäisches Patentamt
European Patent Office
Office européen des brevets

(11) EP 3 878 453 A1

(12) EUROPEAN PATENT APPLICATION
published in accordance with Art. 153(4) EPC

(43) Date of publication:
**15.09.2021 Bulletin 2021/37**

(21) Application number: **19881870.0**

(22) Date of filing: **19.09.2019**

(51) Int Cl.:
***A61K 31/566*** *(2006.01)* ***C07J 73/00*** *(2006.01)*
***A61P 35/00*** *(2006.01)* ***A61K 31/138*** *(2006.01)*

(86) International application number:
**PCT/RU2019/000649**

(87) International publication number:
**WO 2020/096485 (14.05.2020 Gazette 2020/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.11.2018 RU 2018139335**

(71) Applicant: **Iliyasova, Natalia Eduardovna**
**Sankt-Peterburg, 197022 (RU)**

(72) Inventors:
- **ILYASOV, Shamil Sionovich**
  **St.Petersburg, 192022 (RU)**
- **SHAVVA, Alexandr Grigorievich**
  **St.Petersburg,192029 (RU)**
- **MOROZKINA, Svetlana Nikolaevna**
  **St.Petersburg,198504 (RU)**

(74) Representative: **KATZAROV S.A.**
**Geneva Business Center**
**12 Avenue des Morgines**
**1213 Petit-Lancy (CH)**

# (54) TREATMENT FOR BREAST CANCER, INCLUDING TRIPLE-NEGATIVE BREAST CANCER

(57) The invention relates to the field of medicine and to the chemical and pharmacological industry, and concerns medicaments for the treatment of breast cancer. In particular the invention relates to a use of 3-O-sulfamoyloxy-7β-methyl-D-homo-6-oxa-8α-estra-1,3,5(10)-tetraen-17a-one as an anti-cancer agent in monotherapy and adjuvant therapy of breast cancer, including the triple negative breast cancer, and a method for the production of the said agent.

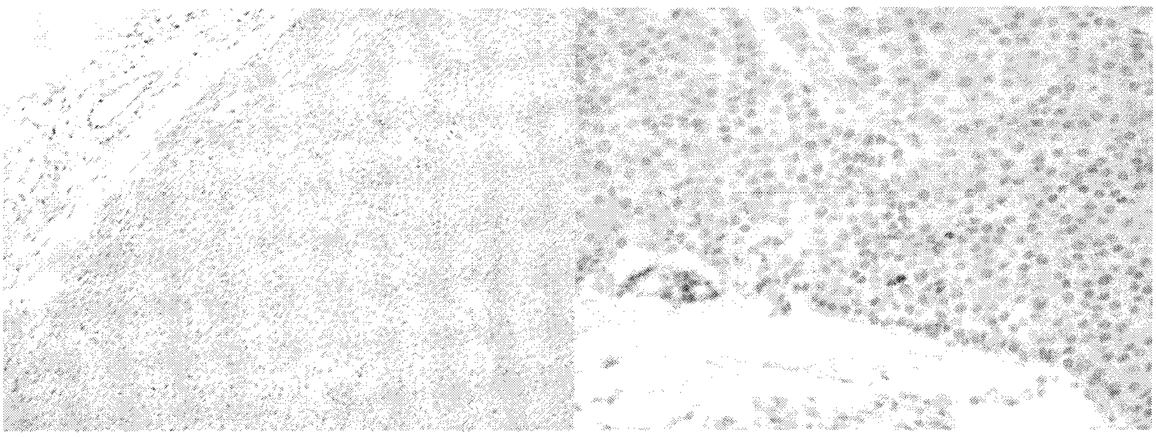

Fig. 1



Printed by Jouve, 75001 PARIS (FR)

## Description

### TECHNICAL FIELD

The present invention relates to the field of medicine and to the chemical and pharmaceutical industry, and concerns medicaments for the treatment of breast cancer.

### BACKGROUND OF THE INVENTION

Breast cancer is the leading oncological disease in women [Parkin D.M., Bray F., Ferlay J., Pisani P., CA Cancer J. Clin., 2005, vol. 55, p. 74-108]. According to the WHO there are 2.09 million patients worldwide with annual breast cancer-related mortality of 627 thousand women every year [http://www.who.int/news-room/fact-sheets/detail/cancer].

A significant portion of tumors with this localization progresses under the action of estrogens [Yue W., Yager J.D., Wang J.-P., Jupe E.R., Santen R.J., Steroids, 2013, vol. 78, p. 161-170.]. Immunohistochemical analysis reveals breast cancers that express receptors for estrogen (ER), progesterone (PR) and HER2NEU protein (which renders the cancer "herceptin sensitivity"). A significant portion of tumors have ERs, PRs and/or HER2NEU 3+. If a certain tumor has no ERs and PRs and is not sensitive to Herceptin (ER0, PR0, HER2NEU 0-1), it is considered as a triple negative form (ER-/PR-/HER-2-). This is one of the most lethal forms of breast cancer as it has no targets for the inhibition of its growth. Currently, there are no medicaments for the treatment of this form of cancer in the world.

Estrogens circulate in the blood and accumulate in tumors in the form of sulfates, which are unable to bind to estrogen receptors however after being converted into free hormones they activate tumor growth. Therefore, a promising one is a strategic line of the treatment comprising using inhibitors blocking the formation of this group of free hormones in a tumor.

Tamoxifen is a medicament from the group of selective estrogen receptor modulators, which blocks estrogen effects on hormone-dependent tissues including breast tissue. Tamoxifen is a standard of hormone therapy for premenopausal and postmenopausal women. The most typical side effects, which develop as a result of tamoxifen administration include: an increased risk of venous thrombosis, aggravation of the course of cardiovascular diseases (including angina pectoris attacks), development of endometrial neoplasms (polyps and endometrial cancer), as well as uterine fibroma. Tamoxifen is also hepatotoxic. According to the biopharmaceutical classification system (BCS), developed by Gordon Amidon et al. in 1995, tamoxifen is assigned to the second class representing a medicament with low solubility and high permeability.

Aromatase inhibitors have been shown to be more effective than Tamoxifen. Currently, aromatase inhibitors such as letrozole and anastrazole have been put into use in this field. They are assigned to the first class according to the BCS system, which stands for medicaments with high solubility and high permeability. The side effects of aromatase inhibitors include osteoporosis, hot flashes, and headaches.

In addition to the aforesaid, steroid sulfatase is a subject of great attention due to the local interstitial formation of estrogens from the abundant pool of circulating estrone sulfate. Steroid sulfatase catalyzes the hydrolysis of estrone sulfate to estrone and DHEA sulfate to DHEA (Dibbelt I., Biol. Chem., Hoppe-Seyler, 1991, vol. 372, p. 173-185.; Stein C., J. Biol. Chem., 1989, vol. 264, p. 13865-13872.).

The most known steroid sulfatase inhibitor is EMATE - estrone sulfamate (Ahmed S., Curr. Med. Chem., 2002, vol. 9, no. 2, p. 263-273.). However it has a significant disadvantage. Estrone sulfatase inhibitors having a sulfamate group cause an irreversible enzyme deactivation with the release of a free ligand [Howarth N.M., Purohit A., Reed M.J. J. Med. Chem., 1994, vol. 37, p. 219-221.]. In particular, estrone sulfamate inhibits estrone sulfatase but the release of free hormone leads to the emergence of strong uterotropic activity [Shields-Botella J. et al., J. Steroid Biochem. Mol. Biol., 2003, vol. 84, p. 327-335.].

A group of estrone sulfatase inhibitors are described in WO9933858 (A2) -1999-07-08, wherein the derivatives of the following general formula can be used as estrone sulfatase inhibitors for the treatment or prevention of estrogen-dependent disorders, such as breast cancer:

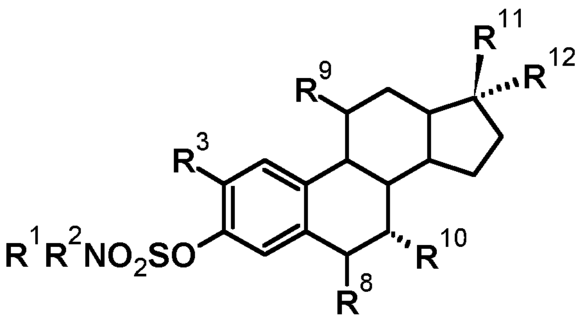

These compounds have uterotropic activity as well.

Therefore when searching for new anti-cancer agents it is necessary to take into account that a carrier of the sulfamate group of the estrone sulfatase inhibitor should not possess hormonal activity.

These requirements led to the expansion of the applications of estrogen-based sulphamates in view of their wide range of activity, in particular, their use against recurrent breast cancer [Shah R., et al., Sulfatase inhibitors for recidivist breast cancer treatment: A chemical review, Eur. J. Med. Chem., 2016, vol. 114, p. 170-190.].

The prior art also discloses a compound 3,17αβ-disulfamoyloxy-7β-methyl-D-homo-6-oxaestra-1,3,5(10), 8,14-pentaen of the formula

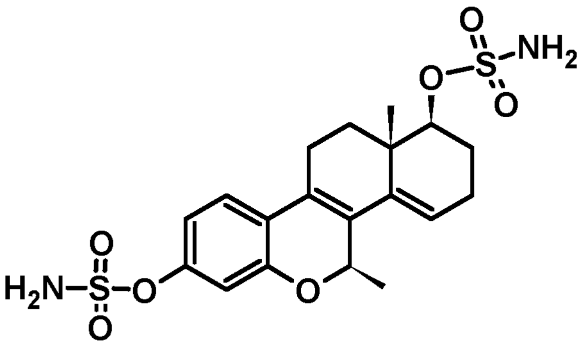

as an inhibitor of the growth of MCF-7 breast cancer cells (RU 2619457, published 16.05.2017). This compound can be selected as the closest prior art to the present invention.

**DESCRIPTION**

A technical problem of the present invention is to provide compounds and develop an efficient method of synthesis thereof, which compounds can be used as anti-cancer agents in monotherapy and adjuvant therapy of breast cancer, including its most lethal form such as triple negative breast cancer (ER-/PR-/HER-2-).

The problem is solved by providing a use of the compound of formula **(1)** named 3-O-sulfamoyloxy-7β-methyl-O-homo-6-oxaestra-1,3,5(10),8(9)-tetraen-17a-one inhibiting estrone sulfatase

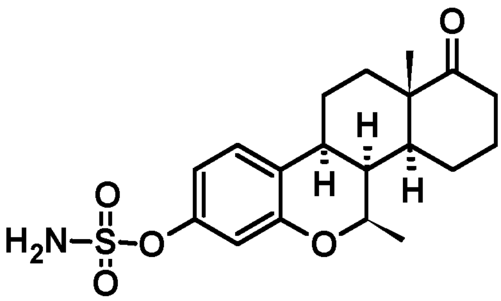

1,

as an anti-cancer agent in monotherapy and adjuvant therapy of initial and progressive forms of breast cancer, including

the triple negative form (ER-/PR-/HER-2-) of breast cancer.

The above-mentioned problem is also solved by providing a process for the preparation of an anti-cancer agent. A distinguishing advantage of the proposed scheme is that it allows for scaling up the target compound production to industrial scale. The process involves acidic hydrolysis of 7β-methyl-6-oxa-D-homo-8α-estrone methyl ether by hydrobromic acid in acetic acid, with the hydrolysis carried out under an argon atmosphere, followed by ethyl acetate extraction, washing with water, saturated sodium chloride solution, drying; the resulting racemic 7β-methyl-6-oxa-D-homo-8α-estrone is dissolved in dimethyl formamide followed by the addition of sulfamoyl chloride; the reaction product is extracted with ethyl acetate, washed with saturated sodium chloride solution, dried with sodium sulphate.

The resulting anti-cancer agent belongs to the 5th hazard category ($LD_{50}$> 5000 mg/kg). The obtained data allow ranking the doses for the study of antitumor activity as safe. Therapeutically effective doses of the medicament are preferably from 0.1 to 20 mg/kg.

Breast tumors (BT) in female FBV/N mice transgenic for HER-2/neu are adenocarcinomas characterized by a low amount of estrogen receptors (ER) (Fig. 1) and a lack of progesterone receptors (PR). Two adenocarcinoma subtypes can be differentiated, in particular ER+, PR-, and ER-, PR- [2]. The immunohistochemical analysis of ERa in breast tumors has revealed single positively stained nuclei only in 10 out of 45 samples (Fig. 1).

Figure 1 depicts the staining of breast tumor with ERa antibodies. Visualization: horseradish peroxidase + diaminobenzidine, magn. x100 (A). The specific staining of cell nuclei of breast duct and nuclei of individual tumor cells (B), magn. x400.

The model is sensitive to the CAF therapy (cyclophosphamide, adriamycin, 5-fluorouracil) with the observable tumor growth inhibition (TGI) resulting in the stabilization of the average tumor volumes (Fig. 2). The TGI was 63% on Day 14 and 46% on Day 21 of the experiment.

Figure 2 depicts the average volume of tumors established at the beginning of the experiment in mice receiving the CAF therapy.

As animals may develop multiple tumors, it is considered reasonable to analyze an average total tumor volume in mice (Fig. 3) and its relative change versus tumor volume at the beginning of the experiment (Fig. 4). The graph of the relative increase in tumor volume shows a linear change of this parameter in the control group suggesting that this parameter is appropriate to use for assessing a biological response of tumor to the treatment. For instance the treatment with the CAF therapy does not cause any changes in this parameter for almost 3 weeks indicating disease stabilization. The frequency of individual tumor stabilizations is about 60% by Day 21.

The study was carried out in female FVB mice transgenic for HER-2/neu by conventional category; the strain was obtained from the mouse bank Charles River Laboratories (Italy). At the beginning of the study the animals were 21-41 weeks old having weight of 25 to 30 g.

Tamoxifen Hexal (Hexal AG, Germany, 83607 Holzkirchen, lot HA1575 on 01/17) representing a dosage form of the medicament formulated as white or slightly yellowish coated round biconvex tablets, with uniform smooth surface, comprising 20 mg of the active compound was used for the experiment.

The housing conditions were chosen according to the standards specified in The Guide for Care and Use of Laboratory Animals (ILAR publication, 1996, National Academy Press, 1996).

The animals were housed in a separate room in groups (no more than 5 animals in a group) in individual T2 type cages suitable for laboratory rodents. The dimensions of the cages were 268 x 215 x 141 $mm^3$ (base area 370 $cm^2$) each equipped with a polycarbonate tub, stainless steel lid with a feed container, and divider for drinking bottle. The cage bedding was made of dust-free wood shavings.

The animals had unrestricted access to the combined complete pelleted feed for laboratory rodents.

Drinking water was given *ad libitum* in standard 190 ml drinking bottles manufactured by TECNIPLAST, made of high-temperature polysulfone with a silicone ring and a metal lid made of AISI 316 stainless steel.

The room temperature was maintained at a level of 20-26°C with the relative humidity of 50-70%. The photoperiod was set at 12:12 hrs night/day under artificial lighting with fluorescent lamps.

An identification card was placed on the housing cage with a cage/card number, experimental group number, individual numbers of the animals, their number and sex, study number, name of the responsible researcher. The auricle of each animal was clipped with a metal marker for small laboratory rodents made of Kent-Scientific nickel alloy, USA (markers have three-digit numbers stamped by the manufacturer).

The mice were euthanized with carbon dioxide at the end of the experiment. All animal corpses were autopsied followed by microscopic description.

$LD_{50}$ of the medicament was determined in FVB mice transgenic for HER-2/neu using the OECD 423 test (OECD guideline for testing of chemicals. Acute Oral Toxicity - Acute Toxic Class Method).

12 female mice were used in the experiment.

At the first step, 3 mice were given the medicament at a dose of 300 mg/kg. The study medicament was administered intragastrically (i.g.) using a metal atraumatic gavage (the procedure corresponds to the oral administration in clinical settings). For i.g. administration the active compound of the medicament was mixed with olive oil to prepare

the suspension of the required concentration for the administration 0.1 ml of the suspension per 10 g of an animal body weight. The dosage form for administration was prepared *ex tempore.*

As no animals died after administration of the medicament at a dose of 300 mg/kg, the same dose was administered to 3 additional animals. No deaths were detected either. Therefore, the medicament was further administered at the dose of 2000 mg/kg in 2 steps, 3 animals per each step.

The clinical observation and registration of body weight were made according to the scheme as shown in Table 1. The animals were examined 2 times a day, clinically examined individually after the dose administration during the first 60 minutes, with special attention during the first 4 hours, periodically during the first 24 hours, on Day 2 and weekly for up to 14 days. Each animal was thoroughly examined in the housing cage in the observer's hands. The overall conditions of the animals were recorded, such as peculiarities in their behavior, intensity and nature of motor activity, fur and skin appearance.

The body weights of the animals were recorded using a verified high-speed electronic laboratory balance Ohaus Scout Pro (USA) with a maximum load of 2000 g and a measurement step of 0.1 g.

On Day 15, the animals were euthanized and undergone autopsy analysis with the registration of macroscopic changes. The data was collected in a special individual autopsy form.

All of the experimental animals were subject to pathomorphological examination at the end of the study. The euthanized animals were carefully examined for the external pathological changes. The chest and abdominal cavities were examined along with the macroscopic analysis of internal organs. The microscopic analysis of organs and tissues of laboratory animals was not carried out, because no deaths of animals were recorded over the whole observation period. In addition, the macroscopic analysis of the internal organs did not reveal any pathological changes.

Table 1.

| Scheme of observation and weighing of animals when assessing acute toxicity | | | | | | | | | | | | | | | |
|---|---|---|---|---|---|---|---|---|---|---|---|---|---|---|---|
| Parameters | Day of experiment (weighing on Day 1 is conducted before the medicament administration) | | | | | | | | | | | | | | |
| | 1 | 2 | 3 | 4 | 5 | 6 | 7 | 8 | 9 | 10 | 11 | 12 | 13 | 14 | 15 |
| Weighing | | | | | | | | | | | | | | | |
| Administration of medicaments | | | | | | | | | | | | | | | |
| Clinical examination | | | | | | | | | | | | | | | |
| Euthanasia of animals | | | | | | | | | | | | | | | |

The study medicament was administered i.g. using a metal atraumatic gavage (with the procedure corresponds to the oral route of administration in clinical settings). The medicament was administered in a single daily dose (20 mg/kg of body weight) and 5-fold daily dose of 100 mg/kg of body weight. *Ex tempore* solutions of the medicament for administration were prepared in olive oil (refined olive oil supplemented with unrefined extra virgin olive oil available under Global Village "Clasico" trademark, lot L:183351116, expiry date 26.04.2020, BAIEO, Spain). The administered volume was 0.1 ml per 10 g of a mouse body weight (0.2 ml of a ready-to-use formulation for a mouse of 20 g). The medicament administration was started 24 hours after the randomization. The duration of the administration course was 27-28 days.

The reference drug, Tamoxifen, was also administered using a metal atraumatic gavage (the procedure corresponds to the oral administration in clinical settings), at a daily dose of 4.0 mg/kg calculated on the basis of the clinical doses [5]. This dose corresponds to a human daily dose of 20 mg/kg. A tamoxifen tablet was crushed in a pounder, and the resulting powder was used to prepare a suspension in 40 ml of the olive oil, the administered volume was 0.08 ml per 10 g of a mouse body weight, the duration of administration was the same as that of the experimental medicament and last for 27-28 days.

The control groups were given the olive oil (placebo).

The evaluation criteria included the clinical observation data, animals body weights, time of death (if applicable), tumor growth over time, pathomorphological examination data (verification of a neoplasm during autopsy, assessment of toxic effects by macroscopic presentation of changes in the internal organs).

The animals body weights were recorded before the first administration of the medicaments and then twice per week using a verified high-speed electronic laboratory balance Ohaus Scout Pro (USA) with a maximum load of 2000 g and a measurement step of 0.1 g.

Macroscopically detectable tumor nodes were measured in animals once per week during weighing process. Two linear dimensions including the largest one and the largest rectangular to the former were recorded for each of the

nodes. The largest dimension was taken as a length (a) and the second dimension as a width (b) of the tumor nodes. A tumor volume was calculated by the expression as follows:

$$V=(a\times b)2/2,$$

The efficacy of the therapy was assessed by recording a change in the average tumor volume, tumor growth inhibition (TGI), and the average total tumor volumes and its change over time in each of the mice.

The percent of tumor growth inhibition was calculated by the expression as follows:

$$TGI=(V_{ctrl}-V_{exp})/V_{ctrl}\times 100\ (\%),$$

where $V_{ctrl}$ is an average tumor volume in a control group, and $V_{exp}$ is an average tumor volume in an experimental group.

The primary data from individual forms were transferred to Microsoft Excel 2007 books. The group arithmetic average (M) and standard deviation (m) were calculated for all quantitative data. The statistical analysis was performed using the statistical software GraphPad Prism 6.0. The differences between the groups were assessed using ANOVA regression analysis and the Fisher's exact test.

When assessing the acute toxicity the animals were continuously observed for the first 60 minutes, and then examined every hour for 3 hours and further once in 24 hours. On the second day, the observation was carried out twice per day. Further the observations were made once per day. The clinical examination of each of the animals was carried out after the active compound administration, every other day, and then weekly. The animal was thoroughly examined in the housing cage in the observer's hands. The overall conditions of the animals were recorded, such as peculiarities in their behavior, intensity and nature of motor activity, fur and skin appearance.

During the experiment no animals deaths were detected when the medicament was administered at a dose of 300 mg/kg. None of the animals exhibited visual signs of intoxication over the entire observation period. The appearance and behavior of the animals were as usual. When picked up by hands, the animals displayed a conventional weak reaction. No vocalization from the animals was detected upon the administration of the medicament or shortly thereafter.

The daily monitoring of the general condition and behavioral reactions of the animals has shown that a single oral administration of the test medicament did not affect the general condition and activity of the experimental animals exposed to intoxication.

The body weights are shown in Tables 2 and 3.

Table 2.

| Changes in the body weights of the animals upon administration of the medicament at a dose of 300 mg/kg | | | | |
|---|---|---|---|---|
| Dose of 300 mg/kg | Days of experiment | | | |
| | 1 | 2 | 7 | 14 |
| Series 1 | 03.05.2018 | 4.05.2018 | 9.05.2018 | 16.05.2018 |
| Mouse No. 1 | 29.0 | 29.2 | 29.6 | 29.8 |
| Mouse No. 2 | 26.4 | 26.4 | 27.0 | 27.2 |
| Mouse No. 3 | 28.2 | 28.0 | 28.4 | 28.6 |
| Series 2 | 15.05.2018 | 16.05.2018 | 21.05.2018 | 28.05.2018 |
| Mouse No. 4 | 30.0 | 30.8 | 30.8 | 30.3 |
| Mouse No. 5 | 29.0 | 29.6 | 31.2 | 31.0 |
| Mouse No. 6 | 29.5 | 28.6 | 30.4 | 30.6 |
| Average (% from baseline) | 28.7 | 28.8 (0) | 29.6(3) | 29.6(3) |
| Lethal effects (dead/total) | | | 0/6 | |

Table 3.

| Changes in the body weights of the animals upon administration of the medicament at a dose of 2000 mg/kg | | | | |
|---|---|---|---|---|
| Dose of 300 mg/kg | Days of experiment | | | |
| | 1 | 2 | 7 | 14 |
| Series 1 | 30.05.2018 | 31.05.2018 | 05.06.2018 | 12.06.2018 |
| Mouse No. 1 | 26.2 | 24.7 | 25.7 | 26.5 |
| Series 1 | 17.09.2018 | 18.09.2018 | 23.09.2018 | 30.09.2018 |
| Mouse No. 2 | 26.0 | 25.7 | 25.0 | 25.1 |
| Mouse No. 3 | 28.4 | 27.7 | 25.8 | 28.3 |
| Series 2 | 30.09.2018 | 1.10.2018 | 6.10.2018 | 13.10.2018 |
| Mouse No. 4 | 27.7 | 27.0 | 29.6 | 28.0 |
| Mouse No. 5 | 26.2 | 25.3 | 24.6 | 25.9 |
| Mouse No. 6 | 28.3 | 27.4 | 27.6 | 29.1 |
| Average (% from baseline) | 27.1 | 26.3 (-3) | 25.9 (-4) | 27.2 (0) |
| Lethal effects (dead/total) | | | | 0/6 |

As no animals died upon administration of a dose of 300 mg/kg, the administration of the medicament was set at a dose of 2000 mg/kg. The medicament was administered in 2 steps with a break of 3 hours in a volume of 0.1 ml per 10 g of a body weight per step at the concentration of 100 mg/ml, because the suspension in oil is thicker at higher concentrations and precludes the medicament to be administered through a gavage. No clinical signs of intoxication were observed after the administration of 2000 mg/kg dose. Regardless of the group the width of the palpebral fissure of the animals was almost unchanged during the entire observation period. No pathological discharges from the eyes were detected. The nose was pink, moderately moist without pathological discharges. The fur of all mice was neat and shiny without bald spots. A 3 % decrease of the animals body weight was registered on Day 2 and a 4 % weight decrease on Day 7 from the initial weight (Table 3). By the end of the observation period the average body weight did not differ from the initial weight.

On Day 15, the animals were euthanized. Upon macroscopic analysis no changes of the internal organs were detected.

The autopsy data were as follows:

The fur of the animals had a neat appearance, was shiny without bald spots. The nutritional status of the animals was satisfactory.

There were no pathological discharges from natural orifices.

The submandibular lymph nodes were rounded, pale pink in color, and moderately dense. The salivary glands were of normal shape, pale yellow in color, and moderately dense.

The peritoneum was smooth and shiny without free fluid in the cavity.

The spleen was not enlarged, dense, and the capsule was smooth and shiny. The pancreas was pale pink in color with lobed structure.

The size and shape of the liver were not changed, the liver capsule is shiny. The liver tissue had brownish color and moderately dense texture.

The kidneys were dense, the capsule was smooth, shiny, and there was a moderate outgrowth of adipose tissue around the kidneys.

The ovaries were not enlarged with shiny surface.

The stomach had folded shiny mucous membrane with a small amount of mucus and food contents in the lumen.

The pleura was smooth and shiny without free fluid in the chest cavity. The thymus was triangular in shape, whitish in color. Lungs were light pink in color, airy.

Therefore, according to the OECD 423 test, the medicament can be assigned to the $5^{th}$ or unclassified category with $LD_{50}$ being equal to or more than 5000 mg/kg, which corresponds to the class of low-hazard compounds according to GOST 12.1.007-76.

The animals satisfactorily tolerated the administration of the medicament, no clinical signs of toxicity being observed. The animal body weights were somewhat decreasing by the end of the first week of the medicament administration. The same holds true for the control group, which was likely due to the stress associated with the oral administration of the medicament. A gain in body weights was observed later, which, might be also attributed to the increase in the volume of tumor nodes in the mice (Table 4).

No specific signs of toxic damage to the internal organs were detected after the autopsy at the end of the experiment. Across all groups of mice, there was fatty infiltration of the wall of the upper small intestine region, which was likely due to oil administration.

Table 4.

| Changes in the average body weight of mice (g) when assessing the antitumor activity of the medicament | | | | | | |
|---|---|---|---|---|---|---|
| Group | Parameter | 27.06.18 (1) | 02.07.18 (6) | 09.07.18 (13) | 16.07.18 (20) | 23.07.18 (27) |
| Control-1 | M | 27.5 | 26.9 | 27.5 | 28.1 | 29.3 |
| | m | 0.8 | 0.7 | 0.8 | 0.9 | 1.0 |
| 20mg/kg | M | 28.9 | 27.5 | 28.2 | 28.8 | 30.2 |
| | m | 0.7 | 0.6 | 0.8 | 1.0 | 1.2 |
| 100 mg/kg | M | 28.6 | 27.6 | 28.3 | 28.6 | 30.3 |
| | m | 0.5 | 0.4 | 0.6 | 0.7 | 0.8 |
| Tamoxifen-1. 4 mg/kg | M | 27.7 | 26.5 | 27.3 | 27.5 | 28.2 |
| | m | 0.8 | 0.8 | 0.9 | 0.9 | 1.0 |

The results on the changes in the average volume of tumor at the start of the experiment are given in Fig. 5 and Table 5.

The average tumor volumes were increasing throughout the observation period across all groups. TGI on Day 27 was 9% and 21% across the 20 mg/kg and 100 mg/kg groups, respectively. This activity is similar to that of the reference drug Tamoxifen, for which TGI by the end of the observation period was 35% ($p<0.05$ as compared to the control group).

Table 5.

| Changes of the average volume of tumors ($cm^3$) at the beginning of the experiment when assessing the medicament's antitumor activity | | | | | | | |
|---|---|---|---|---|---|---|---|
| Group | Parameter | 25.06.2018 (-1) | 02.07.2018 (6) | 09.07.2018 (13) | 16.07.2018 (20) | 23.07.2018 (27) | % of change to the initial value |
| Control-1 (N=23) | M | 0.18 | 0.3 | 0.56 | 0.66 | 0.89 | 394 |
| | m | 0.03 | 0.05 | 0.09 | 0.09 | 0.13 | |
| 20 mg/kg (N=27) | M | 0.27 | 0.32 | 0.39 | 0.6 | 0.81 | 200 |
| | m | 0.05 | 0.06 | 0.08 | 0.13 | 0.16 | |
| | TGI, % | -50 | -7 | 30 | 9 | 9 | |
| 100 mg/kg (N=25) | M | 0.34 | 0.38 | 0.46 | 0.55 | 0.7 | 106 |
| | m | 0.12 | 0.11 | 0.11 | 0.1 | 0.14 | |
| | TGI, % | -89 | -27 | 18 | 17 | 21 | |
| Tamoxifen-1. 4 mg/kg (N =29) | M | 0.23 | 0.26 | 0.3 | 0.47 | 0.58* | 152 |
| | m | 0.07 | 0.07 | 0.07 | 0.1 | 0.11 | |
| | TGI, % | -28 | 13 | 46 | 29 | 3 | |

M is an average value, m is a mean error, N is a number of tumors at the beginning of the treatment included in the analysis.

*- $p<0.05$ as compared to the control group.

The average volume of new tumors developed during the observation, as measured at the end of the observation period, showed no statistically significant differences across the groups, but there was a downward trend of this parameter across the experimental groups (Table 6). Thus, across the Control-1 group, the average volume of new tumors was 0.33±0.08 $cm^3$, while across the 20 mg/kg group it was 0.27±0.05 $cm^3$; across the 100 mg/kg group it was 0.20±0.05 $cm^3$, which is 18% and 39% less than that of the control group, respectively. This parameter was 0.16±0.05 $cm^3$ for the Tamoxifen-1 group, which is 52% less than that of the control group.

There were no statistically significant differences in the measured parameters across the experimental groups and the control group, except for the statistically significant TGI for the Tamoxifen-1 group on Day 27 of the experiment. The tumor growth rate gradually decreased across the groups treated with the medicament at a dose of 100 mg/kg.

Table 6.

| Frequency of tumor stabilizations, number and average volume of new tumors developed by the end of the study while assessing the antitumor activity of the medicament | | | | | |
|---|---|---|---|---|---|
| Group | Number of tumors at the beginning of treatment and frequency of their stabilizations | | Absolute number of new tumors | Average volume of new tumors at the end of the observation period | |
| Control-1 | 23 | 4% | 26 | M | 0.33 |
| | | | | m | 0.08 |
| 20 mg/kg | 27 | 19% | 29 | M | 0.27 |
| | | | | m | 0.05 |
| 100 mg/kg | 25 | 16% | 25 | M | 0.20 |
| | | | | m | 0.05 |
| Tamoxifen-1, 4 mg/kg | 29 | 10% | 21 | M | 0.16 (p=0.0728) |
| | | | | m | 0.05 |

Percentage (%) of disease stabilizations is a parameter similar to the RECIST criterion, reflecting the frequency of tumors, which volume has not increased by more than 20 % by the end of the observation period in relation to the beginning of the treatment.

The average total volume of tumors on Day -1 and Day 6 was larger across the experimental groups to that across the control group, while it was found to decrease on Day 13 up to Day 27 (Table 7, Fig. 6), which confirms an anti-tumor effect of the present medicament at a dose of 100 mg/kg and the reference drug, Tamoxifen.

Table 7.

| The average total volume of tumors ($cm^3$) in mice when assessing the antitumor activity of the medicament | | | | | | | |
|---|---|---|---|---|---|---|---|
| Group | Parameter | 25.06.2018(-1) | 02.07.2018(6) | 09.07.2018 (13) | 16.07.2018 (20) | 23.07.2018 (27) | % of change from baseline |
| Control-1 (N=10) | M | 0.42 | 0.68 | 1.29 | 1.51 | 2.04 | 386 |
| | m | 0.06 | 0.12 | 0.26 | 0.31 | 0.49 | |
| 20 mg/kg (N=10) | M | 0.72 | 0.86 | 1.04 | 1.61 | 2.19 | 204 |
| | m | 0.21 | 0.25 | 0.31 | 0.53 | 0.64 | |
| | TGI, % | -71 | -26 | 19 | -7 | -7 | |
| 100 mg/kg (N=10) | M | 0.86 | 0.94 | 1.15 | 1.36 | 1.74 | 102 |
| | m | 0.27 | 0.27 | 0.28 | 0.29 | 0.41 | |
| | TGI, % | -105 | -38 | 11 | 10 | 15 | |

(continued)

| The average total volume of tumors ($cm^3$) in mice when assessing the antitumor activity of the medicament | | | | | | | |
|---|---|---|---|---|---|---|---|
| Group | Parameter | 25.06.2018(-1) | 02.07.2018(6) | 09.07.2018 (13) | 16.07.2018 (20) | 23.07.2018 (27) | % of change from baseline |
| Tamoxifen-1. 4 mg/kg (N=10) | M | 0.66 | 0.76 | 0.88 | 1.38 | 1.68 | 155 |
| | m | 0.17 | 0.18 | 0.28 | 0.37 | 0.41 | |
| | TGI, % | -57 | -12 | -32 | 9 | 18 | |
| M is an average value, m is a mean error, N is a number of animals involved in the experiment. | | | | | | | |

The assessment of the relative change in the average total tumor volume in mice has demonstrated that this parameter linearly changes across the control group (the Control-1 group) and constantly increases. This parameter was statistically significantly lower for all of the experimental groups, including the group treated with the refenrence drug (Table 8, Fig. 7), which confirms that the present medicament under study modulates an effect on the tumor growth, albeit it did not lead to the decrease of the total number of tumors.

Table 8.

| Relative change of the average total volume of tumors to that of Day - 1 (expressed in %) in mice when assessing the antitumor activity of the medicament | | | | | | |
|---|---|---|---|---|---|---|
| Group | Parameter | 25.06.2018 (-1) | 02.07.2018 (6) | 09.07.2018 (13) | 16.07.2018 (20) | 23.07.2018 (27) |
| Control-1 (N=10) | M | 0 | 68 | 227 | 292 | 407 |
| | m | 0 | 16 | 53 | 70 | 92 |
| 20 mg/kg (N=10) | M | 0 | 20 | 38** | 101** | 212** |
| | M | 0 | 6 | 12 | 27 | 45 |
| 100 mg/kg (N=10) | M | 0 | 26 | 68** | 139* | 198** |
| | m | 0 | 16 | 28 | 63 | 75 |
| Tamoxifen-1. 4 mg/kg # (N=10) | M | 0 | 40 | 80* | 161* | 246* |
| | | | | | | |
| | M | 0 | 18 | 41 | 44 | 73 |
| *, **, *** is p <0.05, p <0.01, p <0.001, respectively, as compared to the control group. | | | | | | |

To estimate the hormonal effect of the medicament, the weight of the uterine body without uterine horns was measured (Table 9). The weight of this organ did not differ from that of the control group, which implies the absence of the medicament hormonal effect on the uterus.

Table 9.

| Uterine body weight and body weight of mice with breast tumors when assessing the antitumor activity of the medicament | | | |
|---|---|---|---|
| Group | Uterine body weight (mg) | Phase of the estrous cycle (number of mice) | Body weight (g) |
| Control-1 | 67±3 | E-8 | 28.5±1.0 |
| | 316±3 | D-2 | 32.9±2.5 |
| | 60±6 | All | 29.3±1.0 |

(continued)

| Uterine body weight and body weight of mice with breast tumors when assessing the antitumor activity of the medicament | | | |
|---|---|---|---|
| Group | Uterine body weight (mg) | Phase of the estrous cycle (number of mice) | Body weight (g) |
| Tamoxifen-1, 4 mg/kg | 31±2 | E-9 | 28.0±1.0 |
| | 34 | D-1 | 30.5 |
| | 32±2 | All | 28.2±1.0 |
| 20 mg/kg | 47±7 | E-7 | 30.1±1.7 |
| | 49±16 | D-3 | 30.3±1.8 |
| | 48±5 | All | 30.2±1.2 |
| 100 mg/kg | 56±5 | E-8 | 29.6±0.5 |
| | 26±12 | D-2 | 33.1±4.5 |
| | 50±6 | All | 30.3±0.8 |

The results demonstrate that the medicament is similar to the reference drug in terms of its antitumor activity.

The efficacy of the medicament in terms of TGI tested on a model of multiple breast tumors in FBV/N female mice, transgenic for HER-2/neu was 9% and 21% for the doses of 20 mg/kg and 100 mg/kg, respectively. This activity is similar to that of the reference drug Tamoxifen (4.0 mg/kg), for which the TGI was 35 % by the end of the observation period.

It was shown that the present medicament has no uterotropic activity.

According to the OECD 423 test the medicament can be assigned to the 5th or non-classified category with $LD_{50}$ being equal to or more than 5000 mg/kg, which corresponds to the class of low-hazard compounds according to GOST 12.1.007-76.

## BRIEF DESCRIPTION OF DRAWINGS

Fig. 1 depicts the staining of breast tumor with ERa antibodies.

Visualization: horseradish peroxidase + diaminobenzidine, magn. x100 (A). A specific staining of cell nuclei of mammary duct and nuclei of individual tumor cells (B), magn. x400.

Fig. 2 depicts the average volume of tumors at the beginning of the experiment in mice receiving the CAF therapy.

Fig. 3 depicts the average total volume of tumors at the beginning of the experiment in mice receiving the CAF therapy.

Fig. 4 depicts the relative change in the average total volume of tumors at the beginning of the experiment in mice receiving the CAF therapy.

Fig. 5 depicts the relative change in the average total volume of tumors in mice under estimation of antitumor activity of the medicament at the doses of 20 mg/kg and 100 mg/kg.

Fig. 6 depicts the relative change in the average total volume of tumors in mice when assessing the antitumor activity of the medicament.

Fig. 7 depicts the relative change of the average total volume of tumors in mice when assessing the anti-tumor activity

## EXAMPLES

The invention is further illustrated by examples of carrying out thereof disclosed herein below.

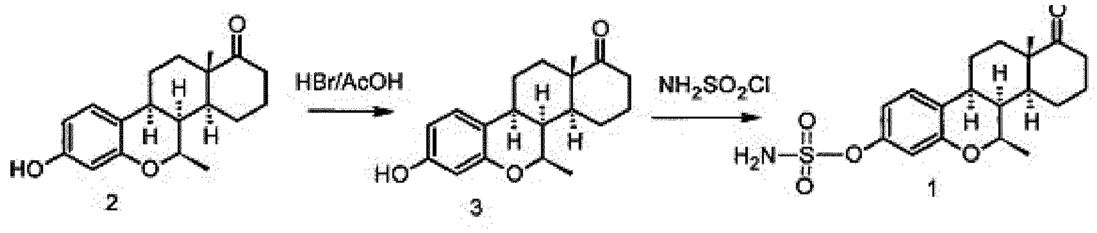


## Example 1.

The synthesis of 3-O-sulphamoyloxy-7β-methyl-D-homo-6-oxa-8α-estra-1,3,5(10)-triene-17a-one of formula **(1).**

To a solution of 300 mg of methyl ether of racemic 7β-methyl-6-oxa-D-homo-8α-estrone of formula **(2)** in 15 ml of glacial acetic acid 2 ml of 40% HBr was added at the room temperature. The resulting reaction mixture was boiled for 7 h, then poured into water, and extracted with ethyl acetate. The organic layers were washed with water, a saturated NaCl solution, and dried over $Na_2SO_4$. The solvent was distilled off on a rotatory evaporator. The yield was 174 mg (62%) of racemic 7β-methyl-D-homo-6-oxa-8α-estrone of formula **(3)** with the melting point of 231-232 °C (the melting point is 230-231°C according to WO2009059806).

Elemental analysis, %: C 76.14; H 8.17. $C_{19}H_{24}O_3$. Calculated, %: C 75.97; H 8.05

To a solution of 100 mg of the compound of formula **(3)** in 1 ml of dimethyl formamide 12 mg of sulphamoyl chloride was added. After 2 hours the reaction mixture was poured into 6 ml of a saturated NaCl solution, and the resulting reaction products were extracted with ethyl acetate. The organic phase was stirred with a saturated NaCl solution followed by drying over sodium sulphate. The yield was 93 mg (74%) of the target product of formula (1), m.p. 195-197 °C. NMR spectrum $^{13}C$ (DMSO-$d^6$), δ, ppm: 214.9, 153.5, 149.8, 125.6, 130.6, 115.0, 111.7, 71.6, 47.4, 44.8, 40.7, 37.9, 34.8, 32.9, 27.4, 26.9, 24.7, 20.0, 19.6. Mass spectrum, m/z ($I_{rel}$, %): 379 (100), 331 (18), 316 (6), 305 (11), 278 (62), 270 (72), 251 (48), 225 (32), 211 (43), 199 (55), 185 (22), 171 (30). Elemental analysis, %: C 60.12; H 6.48; N 3.58. $C_{19}H_{25}NO_5S$. Calculated, %: C 60.14; H 6.64; N 3.69.

## Example 2.

The cells were seeded on Carrel vials at 50x$10^4$ cells per flask. To study the proliferative activity of the cells under conditions of sulfatase inhibition, 24 hours after seeding the culture medium was replaced with the medium containing sulfatase inhibitors to a final concentration of 50 μg/ml, and then these tumor cell lines were incubated for various periods of time (24 to 72 hrs). The inhibitor was dissolved in DMSO. The final concentration of DMSO in the culture medium did not exceed 0.5%. To exclude the cytotoxic effect of DMSO, a control sample comprising DMSO without sulfatase inhibitor was prepared. To exclude nonspecific detrimental effects of the compounds, normal human skin fibroblasts were used. Then the cells were detached with the versene-trypsin solution (Biolot), plated onto Carrel vials containing a fresh complete culture medium. The cells were counted when untreated control cells reached the maximum cell density per unit of surface area of a culture flask (monolayer), and their number was set as 100%. The proliferative activity of all of the studied cell cultures exposed to sulfatase inhibitors was determined in triplicate.

The study of the obtained sulfamate effects on the proliferation of the passaged human MCF-7 cell culture (breast adenocarcinoma) has demonstrated that the compound of formula **(1)** at a concentration of 20 μg/ml completely blocks the proliferation of tumor cells, but it does not influence the growth of human skin fibroblasts having no estrogen receptors. The proliferation of tumor cells is inhibited to the same extent as under the action of Tamoxifen, which has been used in clinical practice for more than 30 years. This is very important because sulfamates and Tamoxifen have different mechanisms of action The experimental data were analyzed with statistical software package "STATISTICA 5.0".

Table 10.

| Effect of the agent on the biochemical parameters of ovariectomized rats | | | | | |
|---|---|---|---|---|---|
| Rat group (number of animals in the group) | Change in body weight during the experiment, g | Uterus weight, mg/100 g bw | Ash femur weight /"Wet" femur weight | Cholesterol level in blood serum, mg/100 ml | Triglyceride level in blood serum, mg/100 ml |
| Sham operated (10) | 29±3* | 200±12** | 0.435±0.005* | 50.4±2.9* | 85.4±5.9* |
| Ovariectomised (15) | 54±4 | 32±3 | 0.400±0.003 | 69.3±3.2 | 69.2±5.4 |

(continued)

| Effect of the agent on the biochemical parameters of ovariectomized rats | | | | | |
|---|---|---|---|---|---|
| Rat group (number of animals in the group) | Change in body weight during the experiment, g | Uterus weight, mg/100 g bw | Ash femur weight /"Wet" femur weight | Cholesterol level in blood serum, mg/100 ml | Triglyceride level in blood serum, mg/100 ml |
| Ovariectomised, treated with EE (0.1 mg/kg bw) (15) | 3±4* | 164±11** | 0.436±0.004* | 45.5±2.1* | 129.8±14.5* |
| Ovariectomised, treated with the medicament (5 mg/kg bw) (15) | 48±4* | 33±3 | 0.390±0.004 | 52.2±2.6* | 59. 59.6±4.8 |
| Note. EE is 17α-ethinyl estradiol.<br>* - P ovariectomized <0.05;<br>** - P ovariectomized <0.01. | | | | | |

The findings demonstrate that the claimed agent has no uterotropic activity, displays a hypocholesteremic action without raising triglyceride levels in the blood serum. The latter is important because high triglyceride levels in serum are associated with a risk of cardiovascular diseases.

In experiments on FVB mice, transgenic for HER-2/neu (ER-/PR-/HER2 +), with breast tumors, the agent inhibits tumor growth to the same extent as clinically approved Tamoxifen, which suggests their use in a combination for the treatment of breast cancer (this might result in lowering of the side effects).

On the MDA-MB-231 line of cells of triple-negative breast cancer the compound inhibits the cell growth, $IC_{50}$ = 4.8 μM, which is similar to Etoposide, a clinically approved chemotherapeutic medicament.

It was demonstrated that the chemotherapy of patients having metastases spread into axillary lymph nodes with the present medicament reliably prolongs recurrence-free survival rate by 11% as compared to the untreated group and increase the overall survival rate as compared to the Tamoxifen treated group.

On animal models with an passaged human tumor of triple-negative breast cancer, the medicament showed a better action as compared to clinically approved Tamoxifen and an aromatase inhibitor such as Letrozole.

## INDUSTRIAL APPLICABILITY

As a result of the conducted experiments it was found that the present compound demonstrates activity against triple-negative breast cancer, is nontoxic, has a hypocholesteremic action, has no effect on triglyceride levels in blood serum, does not adversely affect the endometrium, has no uterotropic action.

In view of its properties mentioned above, the present compound can be used for:

- the monotherapy, and adjuvant therapy for early hormone-positive breast cancer in postmenopausal women,
- the treatment of triple-negative breast cancer,
- the monotherapy and adjuvant therapy of breast cancer in postmenopausal women having been treated with Tamoxifen for 2- or 3-years.
- the treatment for advanced breast cancer at late stages.

## Claims

1. A use of 3-O-sulfamoyloxy-7β-methyl-D-homo-6-oxa-8α-estra-1,3,5(10)-tetraen-17a-one of formula **(1):**

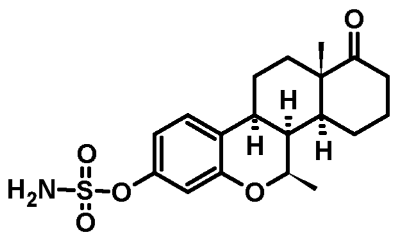

1

as an anti-cancer agent in monotherapy and adjuvant therapy for the treatment of triple-negative breast cancer.

**2.** The use of claim 1, wherein 3-O-sulfamoyloxy-7β-methyl-D-homo-6-oxa-8α-estra-1,3,5(10)-tetraen-17a-one is administered orally to a female patient with early stage or advanced form of a breast cancer, and after the treatment with Tamoxifen for 2- to 3-years.

**3.** A method of preparing an anti-cancer agent as mentioned in claim 1, the method comprising acidic hydrolysis of 7β-methyl-6-oxa-D-homo-8α-estrone methyl ether by hydrobromic acid in acetic acid, **characterized in that** the hydrolysis is carried out under argon atmosphere followed by ethyl acetate extraction, stirring with water and saturated sodium chloride solution, drying; the resulting racemic 7β-methyl-6-oxa-D-homo-8α-estrone being dissolved in dimethyl formamide followed by the addition of sulfamoyl chloride; the reaction product being extracted with ethyl acetate.

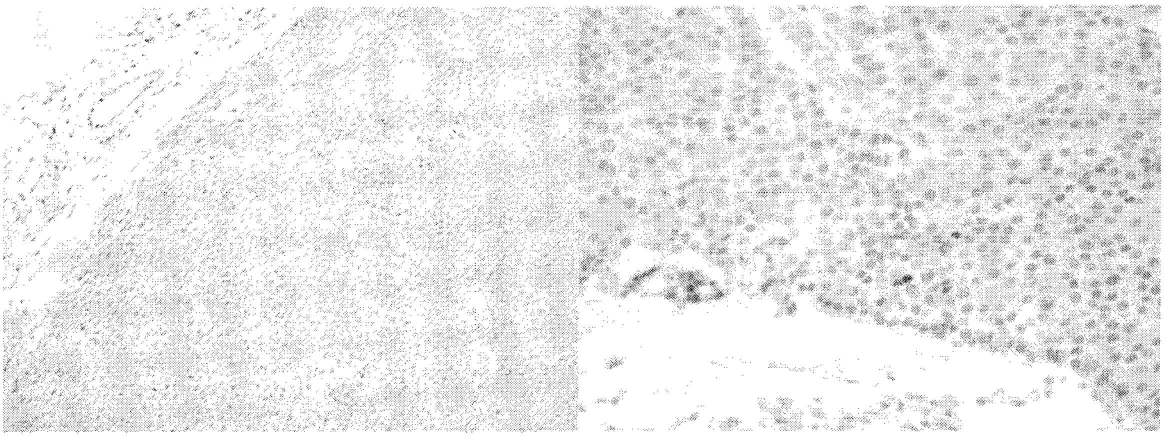

Fig. 1

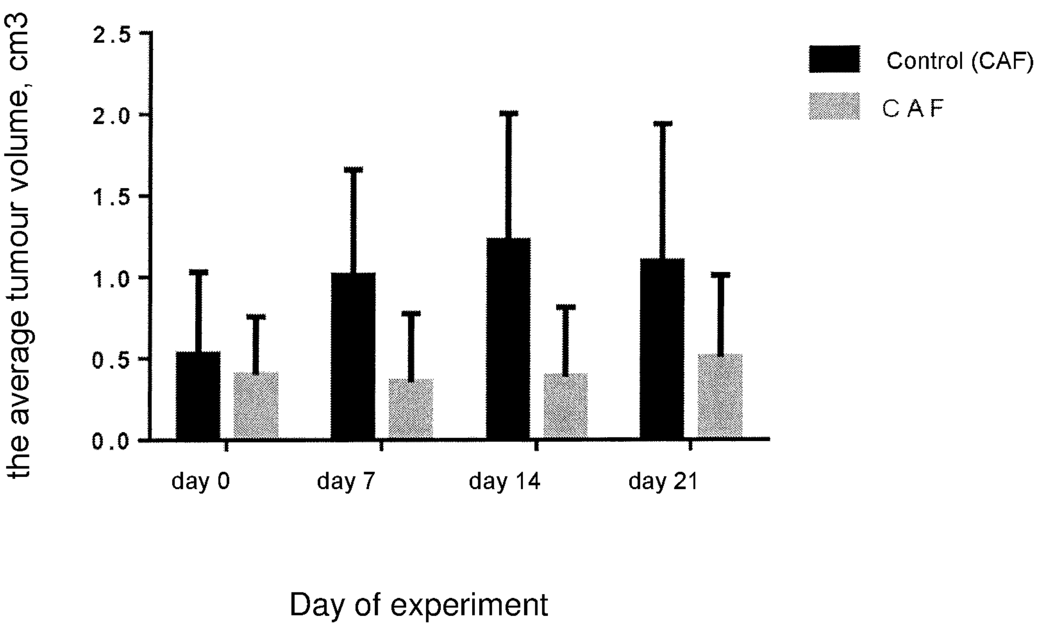
the average tumour volume, cm3
2.5
2.0
1.5
1.0
0.5
0.0
day 0
day 7
day 14
day 21
Control (CAF)
CAF
Day of experiment

Fig. 2

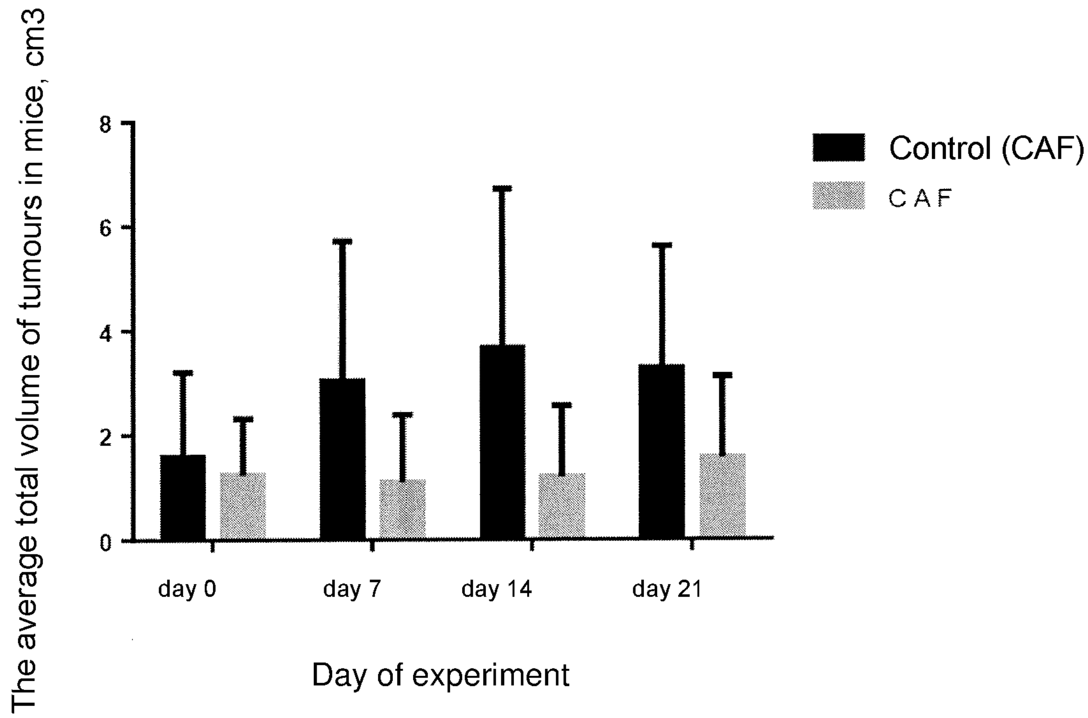
The average total volume of tumours in mice, cm3
8
6
4
2
0
day 0
day 7
day 14
day 21
Day of experiment
Control (CAF)
CAF

Fig. 3

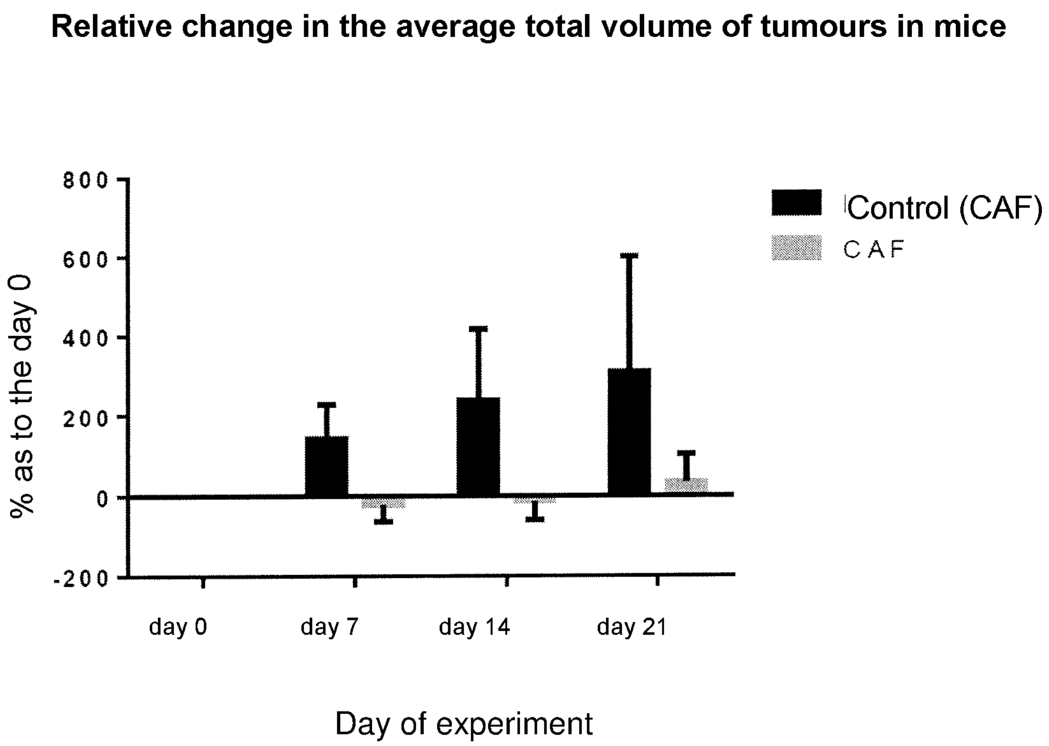
Relative change in the average total volume of tumours in mice
% as to the day 0
800
600
400
200
0
-200
day 0
day 7
day 14
day 21
Day of experiment
Control (CAF)
CAF

Fig. 4

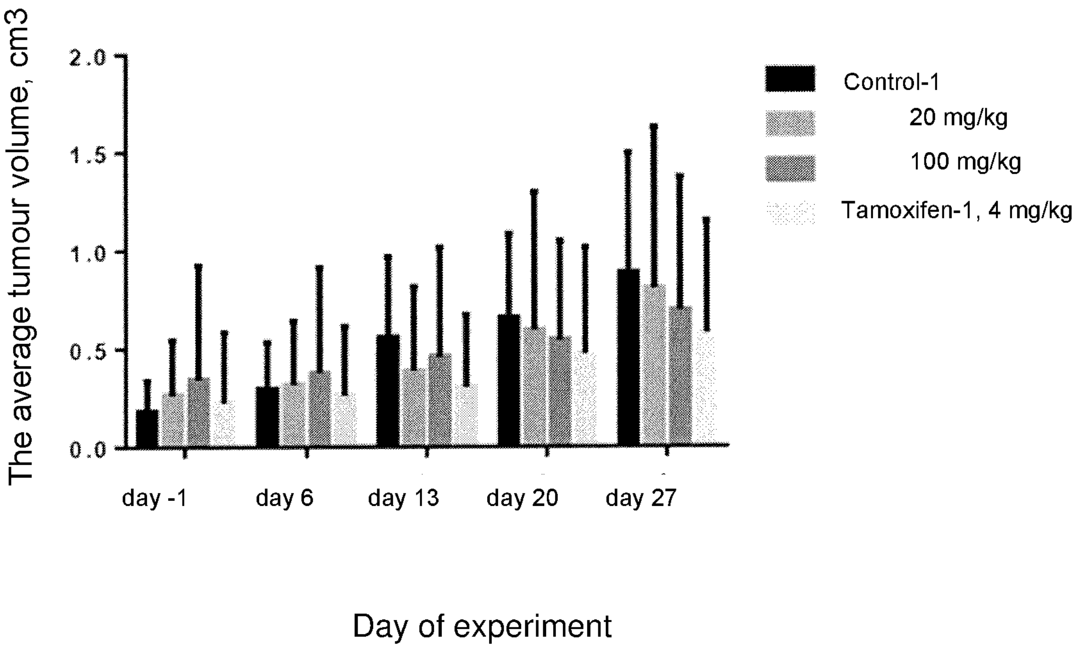
The average tumour volume, cm3
2.0
1.5
1.0
0.5
0.0
day -1
day 6
day 13
day 20
day 27
Day of experiment
Control-1
20 mg/kg
100 mg/kg
Tamoxifen-1, 4 mg/kg

Fig. 5

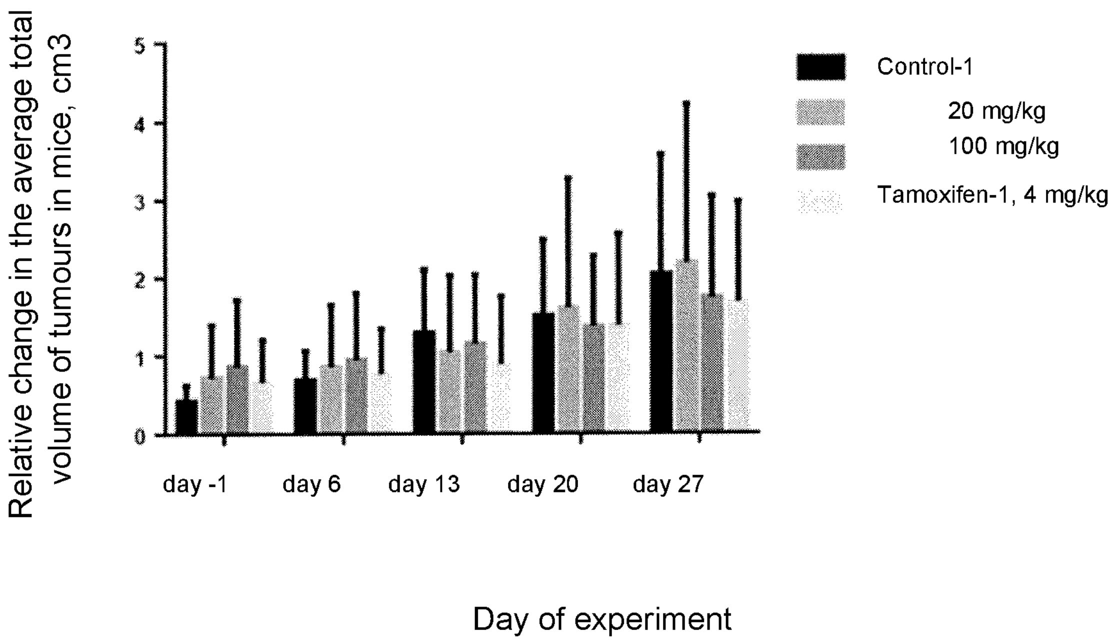
Relative change in the average total volume of tumours in mice, cm3
5
4
3
2
1
0
day -1
day 6
day 13
day 20
day 27
Day of experiment
Control-1
20 mg/kg
100 mg/kg
Tamoxifen-1, 4 mg/kg

Fig. 6

**Relative change in the average total volume of tumours in mice**

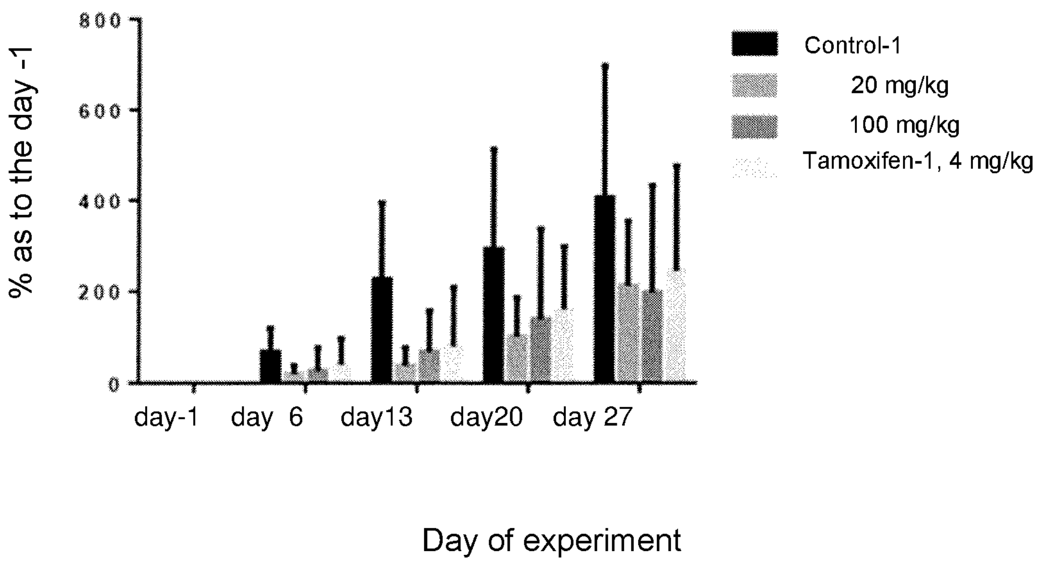


Fig. 7

**INTERNATIONAL SEARCH REPORT**

International application No.
PCT/RU 2019/000649

A. CLASSIFICATION OF SUBJECT MATTER
A61K 31/566 (2006.01); C07J 73/00 (2006.01); A61P 35/00 (2012.01); A61K 31/138 (2006.01)

According to International Patent Classification (IPC) or to both national classification and IPC

B. FIELDS SEARCHED

Minimum documentation searched (classification system followed by classification symbols)
A61K 31/566, 31/138, C07J 73/00, A61P 35/00

Documentation searched other than minimum documentation to the extent that such documents are included in the fields searched

Electronic data base consulted during the international search (name of data base and, where practicable, search terms used)
PatSearch (RUPTO Internal), USPTO, PAJ, Espacenet, Information Retrieval System of FIPS, Google

C. DOCUMENTS CONSIDERED TO BE RELEVANT

| Category* | Citation of document, with indication, where appropriate, of the relevant passages | Relevant to claim No. |
|---|---|---|
| A | US 2018/0085348 A1 (THE REGENTS OF THE UNIVERSITY OF CALIFORNIA) 29.03.2018, items 1, 7, 10 of the claims, table 1 | 1-3 |
| D, A | RU 2619457 C1 (FEDERALNOE GOSUDARSTVENNOE AVTONOMNOE OBRAZOVATELNOE UCHREZHDENIE VYSSHEGO OBRAZOVANIYA "DALNEVOSTOCHNY FEDERALNY UNIVERSITET") 16.05.2017, the abstract, the claims, p. 3, lines 22-35 | 1-3 |
| A | RU 2620084 C1 (FEDERALNOE GOSUDARSTVENNOE AVTONOMNOE OBRAZOVATELNOE UCHREZHDENIE VYSSHEGO OBRAZOVANIYA "DALNEVOSTOCHNY FEDERALNY UNIVERSITET") 23.05.2017, the abstract, p. 3, lines 33-44 | 1-3 |
| A | MOROZKINA S.N. iet al. Sintez i issledovanie nekotorykh biologicheskikh svoistv sulfamatov 8a-analogov steroidnykh estrogenov. Zhurnal organicheskoi khimii, 2015, Volume 51, issue 3, p. 425-430, compound 4c | 1-3 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 December 2019 (23.12.2019) | 30 January 2020 (30.01.2020) |
| Name and mailing address of the ISA/ RU | Authorized officer |
| Facsimile No. | Telephone No. |

Form PCT/ISA/210 (second sheet) (April 2005)

**INTERNATIONAL SEARCH REPORT**

International application No.
PCT/RU 2019/000649

C (Continuation). DOCUMENTS CONSIDERED TO BE RELEVANT

| Category* | Citation of document, with indication, where appropriate, of the relevant passages | Relevant to claim No. |
|---|---|---|
| A | RU 2629186 C1 (FEDERALNOE GOSUDARSTVENNOE AVTONOMNOE OBRAZOVATELNOE UCHREZHDENIE VYSSHEGO OBRAZOVANIYA "DALNEVOSTOCHNY FEDERALNY UNIVERSITET") 25.08.2017, the examples, the abstract | 1-3 |
| A | US 2015/0366824 A1 (THE CURATORS OF THE UNIVERSITY OF MISSOURI) 24.12.2015, paragraphs [0028] - [0036], [0254] | 1-3 |
| A | CN 108379568 A (ACAD OF MILITARY MEDICAL SCIENCES PEA CHINA) 10.08.2018, the abstract | 1-3 |
| A | CH 614217 A5 (SCHERING AKTIENGESELLSCHAFT) 15.11.1979, the claims | 1-3 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9933858 A2
- RU 2619457
- WO 2009059806 A


### Non-patent literature cited in the description


- **PARKIN D.M. ; BRAY F. ; FERLAY J. ; PISANI P.** *CA Cancer J. Clin.,* 2005, vol. 55, 74-108
- **YUE W. ; YAGER J.D. ; WANG J.-P. ; JUPE E.R. ; SANTEN R.J.** *Steroids,* 2013, vol. 78, 161-170
- **DIBBELT I.** Biol. Chem. 1991, vol. 372, 173-185
- **STEIN C.** *J. Biol. Chem.,* 1989, vol. 264, 13865-13872
- **AHMED S.** *Curr. Med. Chem.,* 2002, vol. 9 (2), 263-273
- **HOWARTH N.M. ; PUROHIT A. ; REED M.J.** *J. Med. Chem.,* 1994, vol. 37, 219-221
- **SHIELDS-BOTELLA J. et al.** *J. Steroid Biochem. Mol. Biol.,* 2003, vol. 84, 327-335
- **SHAH R. et al.** Sulfatase inhibitors for recidivist breast cancer treatment: A chemical review. *Eur. J. Med. Chem.,* 2016, vol. 114, 170-190
- Tamoxifen Hexal. Hexal AG
- The Guide for Care and Use of Laboratory Animals. ILAR publication. National Academy Press, 1996